# EUROPEAN PATENT APPLICATION

(11) **EP 4 497 367 A1**
(43) Date of publication of application: **29.01.2025**
(21) Application number: 23188296.0
(22) Date of filing: 28.07.2023
(51) Int. Cl.: A61B 1/00

(54) **SWITCHING ELEMENT AND ENDOSCOPE**

(71) Applicant: InvaView AG, 9462 Montlingen (CH)
(72) Inventor: WEIGER, Ulrich, 9462 Montlingen (CH)
(74) Representative: Keller Schneider Patent- und Markenanwälte AG

(57) **Abstract**

The invention relates to a switching element (1) for attachment to an optical head (3) of an endoscope (2), in particular a rigid endoscope, and for switching a state of the endoscope (2), wherein the switching element (1) is designed to be removable from the optical head (3) and attachable to the optical head (3), and wherein the switching element (1) comprises a first part (5) of a magnetic coupling which first part (5) is, in an attached state of the switching element (1) to the optical head (3), adapted to magnetically couple with a second part (6) of the magnetic coupling arranged in an interior of the optical head (3). The invention also relates to an endoscope (2) designed for receiving the switching element (1) and comprising an optical head (3) and a shaft (4), wherein the second part (6) of the magnetic coupling is arranged in the interior of the optical head (3), wherein an objective is arranged in the shaft (4), and wherein an outer sheath (13) of the endoscope (2) is hermetically sealed at least in the region of the optical head (3).

## Description

### Field of the invention

The invention relates to a switching element for attachment to an endoscope and to the endoscope for receiving the switching element.

### Prior art

Endoscopes as I<nown in the state of the art for use in medicinal contexts allow users to change properties of the endoscope by various means, for example by providing switching rings for adapting a focus of the endoscope or by providing means for changing an orientation of a swing prism which enlarges a possible field of view of the endoscope when held in a specific position in a cavity of a body to be imaged.

Switching rings, means for changing orientation of a swing prism etc., however, complicate an autoclavation/sterilization process that is usually needed after a use of an endoscope as they typically create a more complicated surface structure and/or cavities and/or entry points for fluids into an interior of the endoscope. While complicated surface structures and/or cavities formed by such surface structures may deteriorate results obtained by autoclavation/sterilization processes, entry points for fluids, e.g., at small gaps between a switching ring and the remaining endoscope, may lead to a damaged endoscope in case liquid flows into the endoscope and damages optical components arranged therein. Switchable endoscopes as known in the prior art therefore require complicated mechanical solutions to ensure sufficient autoclavation/sterilization results while at the same time preventing damage due to such operations which are required for hygienic reasons. These needed solutions, however, make endoscopes also more costly. Furthermore, it limits the list of suitable materials, as it requires materials with material specifications that can withstand the autoclavation/sterilization process.

### Summary of the invention

It is an object of the present invention to provide a switching element and an endoscope which switching element and endoscope mitigate at least some of the disadvantages of switchable endoscopes as known in the prior art.

The solution of this object is defined by the features of claims 1 and 11.

According to a first aspect of the invention, the invention relates to a switching element for attachment to an optical head of an endoscope, in particular a rigid endoscope, and for switching a state of the endoscope. The switching element is designed to be removable from the optical head and attachable to the optical head, and the switching element comprises a first part of a magnetic coupling which first part is, in an attached state of the switching element to the optical head, adapted to magnetically couple with a second part of the magnetic coupling arranged in an interior of the optical head.

The switching element according to the invention is designed for being attached to an endoscope, in particular to an endoscope as used in a medicinal context. The endoscope to which the switching element may be attached comprises an optical head that may alternatively be termed proximal end since the optical head is close to a user of the endoscope when in use. Besides the optical head, the endoscope may comprise a shaft, an optical fiber port and an eyecup port or a camera port. The shaft of the endoscope is typically inserted into a body, e.g. a human or animal body. Illumination light may be supplied to the endoscope via the optical fiber port and leave/enter the endoscope at a distal end of the shaft. As known in the prior art, the endoscope may comprise suitable optical elements/objectives in the shaft and/or in the optical head.

The switching element according to the invention is designed in such a way that it can be removably attached to the optical head of an endoscope. When the switching element is in an attached state, i.e., when the switching element is attached to the optical head, it can therefore be removed again. The switching element may be designed in such a way that it can be only once removed after having been attached, i.e., the switching element may only be suitable for a one-time use by design, or the switching element may be designed in such a way that it can be repeatedly attached and removed from the endoscope.

The switching element according to the invention is designed to switch a state of the endoscope through a magnetic coupling between the switching element and the endoscope. The switching element comprises a first part of the magnetic coupling, and a second part of the magnetic coupling is arranged in the interior of the optical head. The switching element is only (electro)-magnetically, in particular only magnetically, connected with an interior of the optical head of the endoscope; there is no direct mechanical connection between the switching element and the interior of the optical head. By way of the magnetic coupling, a movement of the switching element is transduced into an internal movement in the optical head that in turn changes a state of the endoscope. A state of the endoscope that may be switched by movement of the attached switching element may be related to a focusing of the optical elements in the optical head to create a sharp image, for example. A state of the endoscope that may be switched by movement of the attached switching element may also be related to an orientation of a swing prism arranged in the shaft, which swing prism allows for an enlarged field of view of the endoscope. A state of the endoscope that may be switched by movement of the attached switching element may also be related to changing more general imaging properties of an objective in the shaft of the endoscope. A state of the endoscope that may be switched by movement of the attached switching element may also be related to a shutter, arranged in the shaft, having at least two operational states being configured to block ray paths selectively. The shutter can comprise e.g. a rotating disc or a shifting mask, and movement of the attached switching element may be transduced into, e.g., movement of the rotating disc or the shifting mask.

Advantageously, the switching of a state of the endoscope with a switching element according to the invention therefore occurs in an essentially contactless manner, i.e., the interior of the optical head is mechanically separated from the attached switching element by an outer sheath which is hermetically sealed at least in the region of the optical head. As the switching element is removable, the used endoscope can be autoclaved/sterilized and cleaned in an advantageous manner after removal of an attached switching element as the outer surface structure of the endoscope is simplified, in turn leading to fewer cavities and/or places which may be difficult to reach and which obstruct an autoclaving/sterilizing process. Further, as no direct mechanical connection between the switching element and the interior of the optical head exists, the endoscope does not comprise possible entry points for autoclaving/sterilizing fluids that need to be sealed. In contrast to known switchable endoscopes from the prior art, according to the invention complicated sealing solutions are therefore not required to prevent entry of autoclaving/sterilizing fluids into the endoscope or into the switching element.

In an embodiment of the switching element according to the invention, the switching element comprises a first switching element portion and a second switching element portion, wherein the switching element is designed such that the first switching element portion and the second switching element portion can be guided separately to the optical head and can be connected to one another, in particular in a form-fit manner, after at least partially enclosing the optical head.

The first switching element portion and the second switching element may form the switching element after connecting them to one another. To attach the switching element to the endoscope, both switching element portions may be guided to the endoscope and connected to one another thereafter. The first switching element portion and the second switching element portion may be fully physically separated before being connected to one another after at least partially enclosing the optical head, or the first switching element portion and the second switching element portion may already be partially connected to one another before being fully connected after at least partially enclosing the optical head, the partial connection in particular being embodied as a film hinge and the full connection in particular being provided by a snap-fit. A solution comprising a film hinge and a snap-fit advantageously allows for a simple attachment and removal of the switching element to/from the endoscope.

The switching element may be designed in such a way that it tightly encloses at least a part of the optical head, while at the same time allowing at least partial freedom of movement of the attached switching element with respect to the optical head. After attachment of the switching element to the optical head, the switching element may still be rotationally and/or translationally movable with respect to the endoscope to which it is attached to induce a switching of a state of the endoscope.

In a further embodiment of the switching element according to the invention, the first switching element portion comprises a first snap-connect portion and the second switching element portion comprises a second snap-connect portion, wherein the first snap-connect portion and the second snap-connect portion together form a snap-lock or a snap-fit, and wherein the first switching element portion and the second switching element portion can be connected to and disconnected from each other with the snap-lock or the snap-fit.

In a further embodiment of the switching element according to the invention, the first part of the magnetic coupling comprises at least two magnets, in particular formed as permanent magnets.

Besides permanent magnets, the at least two magnets may also be formed as electromagnets whose magnetic properties may be controlled by a control of current flow. Permanent magnets may advantageously be used to enable a solution without need for an external power source, while electromagnets may advantageously be used to enable a solution with a more precise control of how an electromagnetic field is set up, potentially allowing for a more precise switching of states of the endoscope compared with permanent magnets.

In a further embodiment of the switching element according to the invention, the first part of the magnetic coupling comprises a first plurality of magnets arranged in the switching element such that the first plurality of magnets are, in the attached state, arranged in uniform angular increments along a first circle, the center of the first circle lying on a central longitudinal axis passing through a shaft of the endoscope.

Through suitably arranged magnets in the optical head of the endoscope, a rotation of first plurality of magnets around the central longitudinal axis may induce a movement of the magnets in the optical head, for example embodied as a rotation of the magnets in the optical head around the central longitudinal axis. The first plurality of magnets may be arranged as an alternating sequence of north and south poles, for example, and the magnets in the optical head may be arranged as an alternating sequence of north and south poles, for example. The induced movement of the magnets in the optical head may be mechanically transduced into a change of state of the endoscope: in case the state is related to an optical focus of the endoscope, for example, optical elements may for example be moved through a direct mechanical connection along the central longitudinal axis to alter focusing properties; the state may also refer to an angle of orientation of a swing prism; the state may also refer to an opening of a shutter; the state may also refer to more general imaging properties of objectives in the shaft and/or in the optical head. Alternatively, the induced movement of the magnets in the optical head may first be detected using sensors arranged in the optical head, for example using acceleration sensors arranged on the magnets in the optical head or using current sensors measuring induced currents in case the magnets in the optical head are embodied as electromagnets, and the detected movement of the magnets in the optical head may be transduced into a movement of optical elements in the optical head using a dedicated engine arranged in the optical head, which dedicated may be powered by an external power source or a battery, for example.

A uniform angular arrangement of the first plurality of magnets along a first circle centered on the central longitudinal axis may advantageously allow for an arbitrarily oriented first placement of the switching element around the optical head, i.e. in a greater degree of freedom of how a user of the endoscope may initially attach the switching element to the optical head of the endoscope. Alternatively, the switching element may also comprise a marking that provides an indication to a user of how to place the switching element on the endoscope initially.

In a further embodiment of the switching element according to the invention, the switching element is configured to extend, when attached, between i) an eyecup port of the endoscope or a camera port of the endoscope and ii) an optical fiber port of the endoscope.

An eyecup through which a user may look may be attached to the eyecup port. Instead of an eyecup, the endoscope may comprise a camera port through which an image sensor of a camera may receive image information on a field of view captured by the endoscope. The eyecup port or the camera port and the optical fiber port may advantageously restrict translational movement of the attached switching element along the central longitudinal axis.

In a further embodiment of the switching element according to the invention, the switching element is configured such that it can be rotated about the optical head in the attached state, and/or the switching element is configured such that it can be guided translationally along the central longitudinal axis in the attached state.

In a further embodiment of the switching element according to the invention, the switching element is designed such that it can be reused and, in particular, sterilized.

The switching element may be autoclavable/sterilizable, i.e., it may be robust to high temperatures and contact with chemical substances as encountered in typical medicinal processes. This way, after a suitable sterilization, the switching element may be reused. Alternatively, the switching element may be designed for one-time use. This way, less strict requirements may be placed on the switching element, i.e., it may not need to be able to withstand high temperatures or aggressive chemicals. Switching elements designed for one-time use may advantageously offer improved hygienic properties compared to multiple-use switching elements as insufficient autoclavation/sterilization results may be avoided by using one-time switching elements.

In a further embodiment of the switching element according to the invention, the switching element consists essentially of biocompatible, in particular bioinert, plastics.

In a further embodiment of the switching element according to the invention, the switching element is formed as a handle piece.

The switching element may be designed in such a way that a user of the endoscope to which the switching element is attached may additionally use the switching element as a handle piece for holding the endoscope.

According to a second aspect of the invention, the invention relates to an endoscope designed for receiving the switching element according to the first aspect of the invention, the endoscope comprising an optical head and a shaft, wherein the second part of the magnetic coupling is arranged in the interior of the optical head, wherein an objective is arranged in the shaft, and wherein an outer sheath of the endoscope is hermetically sealed at least in the region of the optical head.

In an embodiment of the endoscope according to the invention, the second part of the magnetic coupling comprises at least two magnets, in particular formed as permanent magnets.

In a further embodiment of the endoscope according to the invention, the second part of the magnetic coupling comprises a second plurality of magnets arranged in the interior of the optical head such that the second plurality of magnets are disposed in uniform angular increments along a second circle, the center of the second circle being located on the central longitudinal axis passing through the shaft of the endoscope.

In a further embodiment of the endoscope according to the invention, the second plurality of magnets comprises an equal number of magnets as the first plurality of magnets arranged in the switching element according to the first aspect of the invention.

In a further embodiment of the endoscope according to the invention, the endoscope further comprises a) an eyecup port for attaching an eyecup to the endoscope, wherein the eyecup port is designed such that an eyecup attached thereto and in particular consisting essentially of plastic can be removed from the endoscope and in particular is not directly connected to the switching element, or the endoscope further comprises b) a camera port for attaching a camera to the endoscope.

In a further embodiment of the endoscope according to the invention, the eyecup port or the camera port is threaded, or the eyecup port or the camera port is configured to allow an eyecup or a camera to snap onto the eyecup port or the camera port, respectively.

The invention also relates to a system comprising the endoscope according to the second aspect of the present invention and the switching element according to the first aspect of the present invention, wherein the switching element is attached to the endoscope.

### Brief description of drawings

Exemplar embodiments of the invention are disclosed in the description and illustrated by the drawings in which:
- Fig. 1A-1C: shows perspective views of an embodiment of an endoscope with an attached switching element and an eyecup, of an embodiment of an endoscope with an attached eyecup, and of an embodiment of an endoscope;
- Fig. 2: shows a sectional view along a central longitudinal axis of an endoscope with an attached switching element; and
- Fig. 3A-3C: shows two perspective views of a switching element in a closed state and one perspective view of a switching element in an opened state.

### Detailed description of drawings

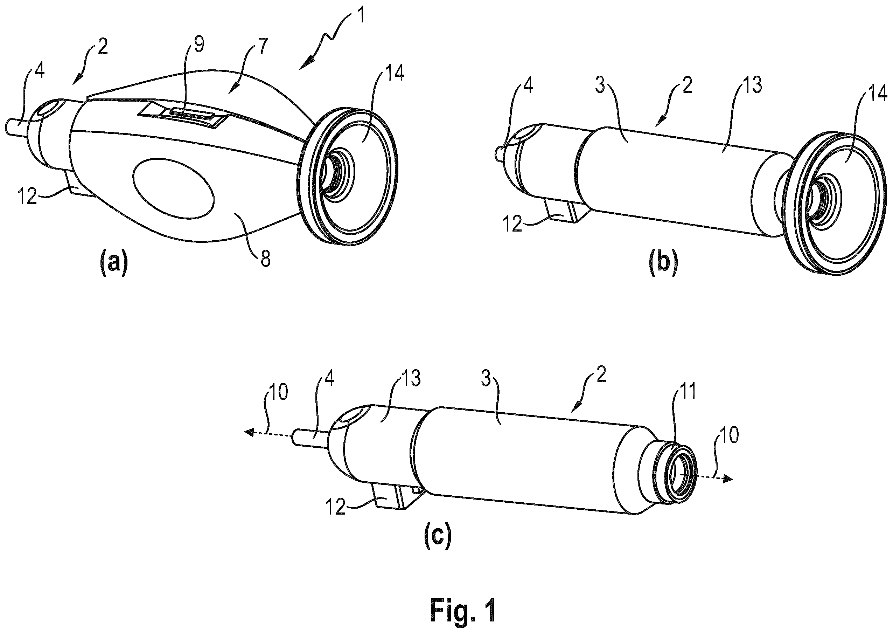

**Fig. 1a** shows an endoscope 2 comprising a shaft 4, an optical fiber port 12, an optical head (covered by an attached switching element 1 in Fig. 1a) and an attached eyecup 14. The switching element 1 comprises a first switching element portion 7 and a second switching element portion 8 which are connected to one another using a snap-fit 9 and a film hinge (not shown in Fig. 1a). Via the snap-fit 9, the first switching element portion 7 and the second switching element portion 8 can be easily disconnected from one another and subsequently removed from the endoscope 2. The eyecup 14 is not directly connected to the switching element 1; the eyecup 14 is attached to the endoscope through a threaded or snap connection.
   The switching element 1 and the endoscope 2 are designed in such a way that when the switching element 1 is attached to the endoscope 2 as shown in Fig. 1a, the switching element 1 can be rotated around the endoscope 2. Via a magnetic coupling (not shown in Fig. 1a) between the switching element 1 and the endoscope 2 and by rotating the switching element 1 around the endoscope 2, a state of the endoscope 2 may be changed.
**Fig. 1b** corresponds to Fig. 1a without an attached switching element. In Fig. 1b, the optical head 3 and an outer sheath 13 of the endoscope are visible. The endoscope 2 of Fig. 1b is such that it is hermetically sealed towards the outside, i.e., substantially no fluids may enter an interior of the optical head 3 in which optical elements sensitive to outside influences are arranged. By comparing Figs. 1a and 1b, it is transparent that when the switching element 1 is attached to the endoscope 2, no direct mechanical connection exists between the interior of the optical head 3 and the switching element 1.
**Fig. 1c** corresponds to Fig. 1b without an attached eyecup. In Fig. 1c, an eyecup port 11 is visible that is used for attaching an eyecup to the endoscope 2. A central longitudinal axis 10 passing through the shaft 4 is shown as well.

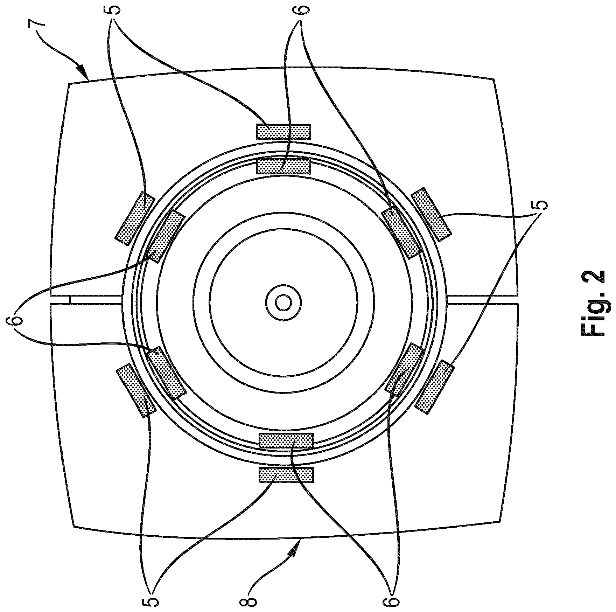

**Fig. 2** shows a sectional view along a central longitudinal axis of an endoscope with an attached switching element. The switching element comprises a first switching element portion 7 and a second switching element portion 8 which are arranged outside the optical head of the endoscope. In Fig. 2, six magnets 5 are shown in the switching element and six magnets 6 are shown in the optical head of the endoscope. By rotating the switching element around the central longitudinal axis, the magnets 6 in the optical head that are movably mounted in the optical head move as well, the movement of the magnets 6 in the optical head in turn leading to a change of a state of the endoscope.

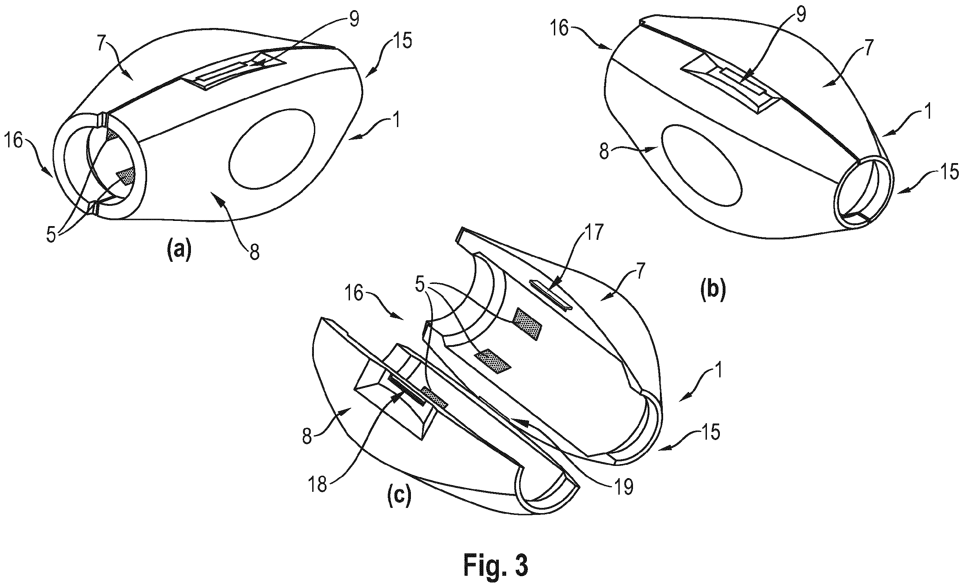

**Fig. 3** shows two perspective views of a switching element in a closed state and one perspective view of a switching element in an opened state.
**In** **Fig. 3a****,** a switching element 1 is shown as view from a first direction. The switching element 1 comprises a first end 15 and a second end 16, wherein when attached to an endoscope, the first end 15 is closer to an eyecup port or camera port of the endoscope than the second end 16 of the switching element 1. The switching element 1 comprises a first switching element portion 7 and a second switching element portion 8 which are connected to one another via a snap-fit 9. In Fig. 3a, two magnets 5 are partly visible, which two magnets 5 form part of the first part of the magnetic coupling.
**Fig. 3b** shows the switching element 1 of Fig. 3a from a different perspective in which the first end 15 of the switching element 1 is in the foreground, and the second end 16 of the switching element is in the background.
**Fig. 3c** shows the switching element 1 in an opened state; the switching element 1 of Fig. 3c is shown from the same viewing direction as the switching element of Fig. 3b. In the opened state, the first switching element portion 7 and the second switching element portion 8 of Fig. 3c are only held together by a film hinge 19.

The first switching element portion 7 comprises a first snap-connect portion 17, and the second switching element portion 8 comprises a second snap-connect portion 18. When the first snap-connect portion 17 and the second snap-connect portion 18 are connected, together they provide the snap-fit 9 as shown in Figs. 3a and 3b. In Fig. 3c, three magnets 5 of the first part of the magnetic coupling are visible as well. When the first switching element portion 7 and the second switching element portion 8 are fully connected to one another using a film hinge 19 on one end and the snap-fit 9 provided by the first snap-connect portion 17 and the second snap-connect portion 18 on the opposite end, the magnets 5 are uniformly spaced along the switching element 1. This way, a user attaching the switching element 1 to an endoscope has greater freedom with respect to an initial placement of the switching element 1 on the endoscope.

In the embodiments of the switching element 1 of Fig. 3, the switching element 1 comprises at both its first end 15 and at its second end 16 inclined end sections which inclined end section are matched to corresponding inclined sections of the endoscope as shown in Fig. 1. The inclined sections of the switching element 1 and of the endoscope are inclined in a complimentary manner. This way, once the switching element 1 of Fig. 3 is attached to the endoscope of Fig. 1, movement of the switching element 1 with respect to the endoscope is substantially restricted to rotational movement around the endoscope, i.e., translational movement of the switching element 1 with respect to the endoscope is substantially suppressed.

To attach the switching element of Fig. 3 to an endoscope, the first switching element portion 7 and the second switching element portion 8 may be guided to the endoscope and may be positioned around the endoscope as shown in Fig. 1. Once the two switching element portions 7, 8 at least partially surround the endoscope, they may be fully connected to one another using the snap-fit 9 provided by the first snap-connect portion 17 and the second snap-connect portion 18.

The switching element 1 of Fig. 3 is structured in such a way that it can be easily handled by a user when attached to an endoscope. The switching element 1 of Fig. 3 in particular comprises a profiled section on a side embodied as a central indentation in the second switching element portion 8. The first switching element portion 7 may comprise a similar central indentation as well.

The switching element 1 of Fig. 3 may be easily autoclaved/sterilized and/or cleaned in case it needs to be used multiple times. Once opened as shown in Fig. 3a, all parts of the switching element 1 are directly accessible from outside and can be reached by a fluid for autoclavation/sterilization and/or cleaning. The switching element 1 of Fig. 3 may therefore be hygienically treated in an advantageous manner after use.

The switching element 1 of Fig. 3 comprises at its second end 16 around its end circumference a protruding section, with around half of the end circumference having a higher profile than the other half. This protruding section may, together with a corresponding stopping element of the endoscope (in Fig. 1c, the corresponding stopping element is displayed to the right of the optical fiber port 12 below the main body of the endoscope), limit rotational movement of the attached switching element with respect to the endoscope. The protruding section of Fig. 3, for example, as it extends around approximately half of the end circumference of the second end 16, limits rotational movement of the attached switching element with respect to the endoscope to a movement of at most around 180° around the endoscope. Based on such a restricted rotational movement of the attached switching element with respect to the endoscope, a simplified mechanical transduction mechanism may be implemented in the endoscope, which mechanical transduction mechanism translates rotational movement of the magnets in the second part of the magnetic coupling to a change of state of the endoscope.

## Claims

1. Switching element (1) for attachment to an optical head (3) of an endoscope (2), in particular a rigid endoscope, and for switching a state of the endoscope (2), wherein
the switching element (1) is designed to be removable from the optical head (3) and attachable to the optical head (3), and wherein
the switching element (1) comprises a first part (5) of a magnetic coupling which first part (5) is, in an attached state of the switching element (1) to the optical head (3), adapted to magnetically couple with a second part (6) of the magnetic coupling arranged in an interior of the optical head (3).

2. The switching element (1) according to claim 1, comprising
a first switching element portion (7) and a second switching element portion (8), wherein
the switching element (1) is designed such that the first switching element portion (7) and the second switching element portion (8) can be guided separately to the optical head (3) and can be connected to one another, in particular in a form-fit manner, after at least partially enclosing the optical head (3).

3. The switching element (1) according to claim 2, wherein
the first switching element portion (7) comprises a first snap-connect portion (17) and the second switching element portion (8) comprises a second snap-connect portion (18), wherein the first snap-connect portion (17) and the second snap-connect portion (18) together form a snap-lock or a snap-fit (9), and wherein
the first switching element portion (7) and the second switching element portion (8) can be connected to and disconnected from each other with the snap-lock or the snap-fit (9).

4. The switching element (1) according to any one of the preceding claims, wherein
the first part (5) of the magnetic coupling comprises at least two magnets, in particular formed as permanent magnets.

5. The switching element (1) according to claim 4, wherein
the first part (5) of the magnetic coupling comprises a first plurality of magnets arranged in the switching element (1) such that the first plurality of magnets are, in the attached state, arranged in uniform angular increments along a first circle, the center of the first circle lying on a central longitudinal axis (10) passing through a shaft (4) of the endoscope (2).

6. The switching element (1) according to any one of the preceding claims, wherein
the switching element (1) is configured to extend, when attached, between
i) an eyecup port (11) of the endoscope (2) or a camera port of the endoscope (2) and
ii) an optical fiber port (12) of the endoscope (2).

7. The switching element (1) according to any one of the preceding claims, wherein
the switching element (1) is configured such that it can be rotated about the optical head (3) in the attached state, and/or wherein the switching element (1) is configured such that it can be guided translationally along the central longitudinal axis (10) in the attached state.

8. The switching element (1) according to any one of the preceding claims, wherein the switching element (1) is designed such that it can be reused and, in particular, sterilized.

9. The switching element (1) according to any one of the preceding claims, wherein the switching element (1) consists essentially of biocompatible, in particular bioinert, plastics.

10. The switching element (1) according to any one of the preceding claims, wherein the switching element (1) is formed as a handle piece.

11. Endoscope (2), designed for receiving the switching element (1) according to any one of the previous claims and
comprising an optical head (3) and a shaft (4), wherein the second part (6) of the magnetic coupling is arranged in the interior of the optical head (3), wherein an objective is arranged in the shaft (4), and wherein an outer sheath (13) of the endoscope (2) is hermetically sealed at least in the region of the optical head (3).

12. The endoscope (2) according to claim 11, wherein the second part (6) of the magnetic coupling comprises at least two magnets, in particular formed as permanent magnets.

13. The endoscope (2) according to claim 12, wherein the second part (6) of the magnetic coupling comprises a second plurality of magnets arranged in the interior of the optical head (3) such that the second plurality of magnets are disposed in uniform angular increments along a second circle, the center of the second circle being located on the central longitudinal axis (10) passing through the shaft (4) of the endoscope (2).

14. The endoscope (2) according to claim 13, wherein the second plurality of magnets comprises an equal number of magnets as the first plurality of magnets arranged in the switching element (1).

15. The endoscope (2) according to any one of claims 11 to 14, further comprising
a) an eyecup port (11) for attaching an eyecup (14) to the endoscope (2), wherein the eyecup port (11) is designed such that an eyecup (14) attached thereto and in particular consisting essentially of plastic can be removed from the endoscope (2) and in particular is not directly connected to the switching element (1), or
b) a camera port for attaching a camera to the endoscope (2).

16. The endoscope (2) according to claim 15, wherein the eyecup port (11) or the camera port is threaded, or wherein the eyecup port (11) or the camera port is configured to allow an eyecup (14) or a camera to snap onto the eyecup port (11) or the camera port, respectively.

17. System comprising the endoscope (2) according to any one of claims 11 to 16 and the switching element (1) according to any one of claims 1 to 10, wherein the switching element (1) is attached to the endoscope (2).
